# EUROPEAN PATENT SPECIFICATION

(11) **EP 1 252 899 B1**
(45) Date of publication and mention of the grant of the patent: **05.07.2006**
(21) Application number: 02425235.5
(22) Date of filing: 17.04.2002
(51) Int. Cl.: A61L 9/03, A01M 1/20, B60H 3/00

(54) **Dispenser of airborne substances**
Spender von Luftschwebestoffen
Distributeur de substances en suspension dans l'air

(30) Priority: 20.04.2001 IT MO20010073
(43) Date of publication of application: 30.10.2002
(73) Proprietor: FALP S.r.l., 40052 Baricella (Bologna) (IT)
(72) Inventor: Falchieri, Roberto., 40126 Bologna. (IT)
(74) Representative: Gotra, Stefano

(56) References cited:
- EP-A- 0 836 857
- WO-A-96/31741
- GB-A- 2 333 233
- US-A- 3 951 622
- US-A- 4 157 787
- US-A- 4 948 047
- US-A- 5 141 707

## Description

Specifically, though not exclusively, the invention can be usefully applied in the field of electric dispensers for diffusing perfumes or insecticides into the surrounding atmosphere.

The invention relates to a device in which the tank containing the volatile substance is of a type having a porous or punctured wall which on one side is in contact with the substance to be diffused and on the other side is in contact with the surrounding atmosphere, into which the vapours are to be dispensed. Patents US-A-4,948,047, US-A-4,157,787 and US-A-3,951,622 teach some examples of containers made in this way.

This type of dispensing device can be distinguished in particular from types having stops, in which the volatile substance in the liquid state is contained in a flagon and the emanation of the vapours occurs through the stop in the container, which projects outward of the container itself and is in contact with the liquid to be dispensed at a bottom of the stop.

The invention relates in particular to a device made according to the preamble of the first claim. EP-A-0836857 teaches a device made in this way, in which the upwards circulation of air is obtained using a ventilator.

The main aim of the present invention is to provide a diffuser in which the upwards circulation of air is achieved in an extremely simple and reliable way. A further aim of the invention is to realise a dispenser which enables an intensity of the vapours dispensed into the air to be regulated, in a manner which is simple, practical and reliable.

An advantage of the invention is to make available a dispensing device which is constructionally simple and economical.

A further advantage is that the vapour released takes only a short time to achieve a good degree of diffusion in the atmosphere.

A still further advantage of the invention is to enable a more-or-less constant emission of vapours into the atmosphere throughout the working life of the product.

These aims and advantages and others besides are all attained by the invention as it is characterised in the claims that follow.

Further characteristics and advantages of the present invention will better emerge from the detailed description that follows of a preferred but non-exclusive embodiment of the invention, illustrated purely by way of nonlimiting example in the accompanying figures of the drawings, in which:
figure 1 is a side view of a container made according to the present invention; figure 2 is a view from the right of figure 1;
figure 3 is section III-III of figure 2;
figure 4 is a side view, partially-sectioned, of an electric dispenser comprising the container of figure 1;
figure 5 is a partial view from the left of figure 4;
figure 6 is a partial view of the electric dispenser of figure 4 in a different operational configuration.

With reference to figures from 1 to 3 of the drawings, 1 denotes in its entirety a container for a volatile substance comprising a closed body which actually contains the substance. The container releases vapours into the air, which are emitted from the volatile substance and which condition the surrounding air. The volatile substance is fluid, preferably liquid, but can also be powder or semiliquid, or a paste or a gel.

The body comprises a flat wall 2 which is permeable to the vapours of the volatile substance, having an edge which is stably coupled, for example by welding, to the edge of an impermeable wall 3. The permeable wall 2 can be porous or perforated. The two coupled walls 2 and 3 delimit a single diffusion chamber which contains the volatile substance; the two walls 2 and 3 also define the capacity of the chamber. The containment and diffusion chamber is not deformable due to the pressure of the volatile substance. At least one of the two walls 2 and 3, preferably the impermeable wall 3, is made of a heat-formable plastic material.

The impermeable wall 3, rigid and not flexible, is conformed so as to exhibit a broadening 4 of the body which in fact constitutes the largest part of the body. This broadening 4 defines a lateral reservoir of the volatile substance which supplies the whole permeable flat wall 2 with the substance.

The container 1 is destined during use to assume a configuration in which the permeable flat wall 2 is vertical and the broadening 4 is located at an upper end of the diffusion chamber and reservoir. In this configuration the volatile substance inside the container is in direct contact with both walls 2 and 3. In effect, the diffusion chamber has a lower part, i.e. destined during use to be located in an inferior position, in which a distance between the permeable wall 2 and the impermeable wall 3 is considerably less than in an upper part of the chamber, in which, due to the effect of the broadening 4, the two walls 2 and 3 are at a much greater distance one from another.

Before use, an impermeable protection film 6, removable at moment of use, is attached to the permeable wall 2. The film 6 can be, for example, peelably coupled to the wall 2 to prevent the volatile substance from exiting before use. The impermeable wall 3 has an end, opposite to the broadening 4, which is flat and facing the permeable wall 2 so as to define a narrow channel 5, full of the volatile substance. In the operational configuration the channel 5 is located below the broadening 4, and communicates there-with. The broadening 4 has a considerable capacity (at least double, but preferably five times or six times greater) in comparison with the underlying channel 5. The chamber therefore comprises an upper storage zone, which extends laterally and which constitutes more or less all of the capacity, and a lower zone, relatively narrow and extended downwards and having only a small capacity, and which supplies the volatile substance to the lowest part containing the permeable wall 2. In other words, the lower part of the body is flattened, while the upper part is considerably broader, particularly on one side thereof.

One or more spacers 51 can be predisposed in the channel 5, interpositioned between the walls 2 and 3 to keep them apart. Each spacer 51 is preferably vertically extended (with reference to the configuration of use of the container) and comprises a rib formed by a recessed part of the impermeable wall 3.

With reference to figures from 4 to 6, 7 denotes in its entirety a dispenser of a volatile substance which will be diffused in the air and activated by a heating process. The dispenser comprises a frame 8 having a housing in which the container 1 as described above will be removably inserted.

The dispenser 7 is provided with a heat source provided by an electrical resistance 9 supplied through a usual plug for connection to the electric supply. The heat source heats up the container 1 and causes evaporation of the volatile substance.

The container 1 is applied on the frame 8 of the dispenser 7 so that during the operation configuration in which the plug is attached to the electricity source, the permeable wall 2 is arranged vertically and facing the plug, while the broadening 4 is situated on an opposite side with regard to the plug.

The dispenser 7 is provided with a wall 10 with no holes or openings and solidly constrained to the dispenser 7 and facing the permeable wall 2 of the body. Between the permeable wall 2 and the wall 10 there is a small chamber 11 which is vertically extended and provided with a lower inlet 12 for air from outside and an upper outlet 13 for air mixed with vapour emitted, through the permeable wall 2, by the volatile substance.

The electrical resistance 9 is predisposed to heat the chamber 11 to cause a circulation of air in the chamber 11, moving upwards from below in a flue effect. To generate the ascending flow of air efficiently, the resistance 9 is located preferably below or near to the lower inlet 12 of the chamber 11.

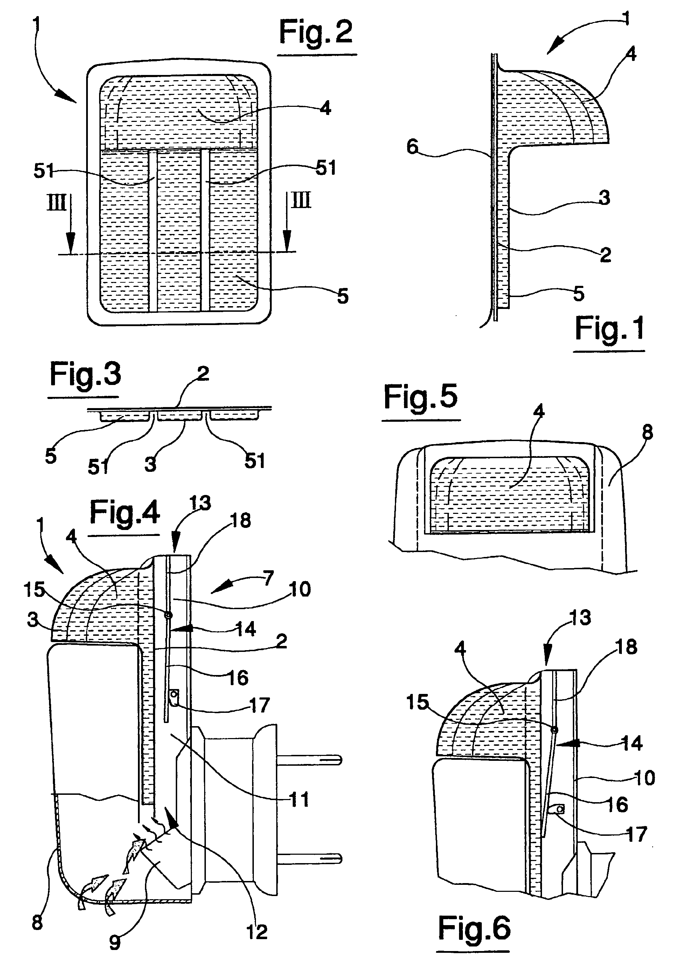

A regulating device 14 is predisposed in the chamber 11 to regulate the quantity of the flow which, rising through the chamber 11, directly strikes the permeable wall 2. In the illustrated embodiment the regulating device 14 comprises a mobile switch 16, rotatable about a hinge 15, which can assume at least one closed position (figure 6), in which it protects at least a part of the permeable wall 2 from the rising air flow, and an open position (figure 4) in which the rising air flow can strike the permeable wall 2 and thus draw with it the vapour issued from the volatile substance. The switch 16 is commanded to close by a cam 17 and returns elastically into the open position.

The regulating device 14 has a fixed upper part 18 which, together with the mobile lower part constituted by the switch 16 vertically divides the chamber 11, or at least a part thereof, into two zones: a first zone faces and is contiguous to the permeable wall 2, and a second zone is separated from the permeable wall 2. The air flow crossing the first zone strikes the permeable wall 2.

The first zone has a lower inlet with an adjustable passage. The rising air circulation passing through the first zone of the chamber 11 contiguous to the permeable wall 2 can be regulated by adjusting the position of the switch 16. In the closed position the rising flow of hot air is deviated almost completely into the second zone of the chamber 11 without touching the first zone, so that it does not strike the upper part of the permeable wall 2 adjacent to the first zone. In this situation of minimum dispensing of vapours to the surrounding atmosphere, the flow rising along the channel strikes only the lower part of the permeable wall 2.

The position of maximum opening, in which the rising flow of hot air is entrained to rise along the first zone of the chamber 11 corresponds to a situation of maximum vapour dispensing. In the intermediate positions the hot air flow is distributed variously to the two zones of the chamber 11.

The greater the quantity of rising flow striking the permeable wall 2 the greater the quantity of vapours from the volatile substance which are diffused into the surrounding atmosphere through the upper exit 13. By adjusting the regulating device 14 the intensity of emanation of vapours can be controlled.

In a further embodiment (not illustrated), the regulating device 14 can be so arranged that in the closed position it protects the whole permeable wall 2 from air circulation, instead of only a part thereof as in the illustrated embodiment. When the container 1 is applied to the dispenser 7, at least a part of the channel 5 is easily inspectable from the outside in order to check on the level of volatile substance remaining. To this end the body is at least partly transparent or translucent.

For the majority of the operative diffusing time, the channel 5 is full and continuously supplied with volatile substance originating from the broadening, which is effectively the reservoir supply substance to the permeable wall 2.

When the upper reservoir is empty, the container is near to empty. As long as the reservoir is even slightly charged, the lower part of the permeable wall, i.e. the part defining the channel 5 and the majority of the permeable wall 2, is in contact with the liquid. Thus the greater part of the permeable wall 2 is always active, i.e. able to diffuse the vapour from the volatile substance for practically the entire working life of the container. Thanks to the position of the upper reservoir, formed by the broadening 4, the intensity of emanation of the vapour is more or less constant from the start to the end of the working life of the container. The invention minimises the variations in intensity of the vapour diffusion during the working life of the container.

In a further embodiment of the container, the permeable wall for diffusing the volatile substance can be other than flat, and the working position can be not vertical but inclined, as long as in any case it exhibits at least an upper part and a lower part. The broadening of the body which forms the supply reservoir is predisposed correspondingly to the upper part of the permeable wall 2.

In a further embodiment, the dispenser is provided with a container, preferably removable, which is constituted by a closed body which contains the volatile substance and which has a permeable wall, through which the vapours of the volatile substance can permeate, stably coupled to an impermeable wall. The body can have (but not necessarily) an upper reservoir formed by a broadening. The dispenser has a wall (similar to the wall 10 of figures 4 and 6) which defines with the permeable wall 2 a chamber (similar to the chamber 11) with an inlet and an outlet for passage of a flow which draws with it the vapour emitted through the permeable wall 2.

In front of the permeable wall 2 there is a regulating device which regulates a quantity of air flow that, crossing the chamber, strikes the permeable wall 2: this device can be, for example, similar to the regulating device 14 as described above.

The vapour drawing flow, which in the above-described embodiment is obtained using means for heating and employing the flue effect, can be obtained in other ways, for example using means for forced ventilation incorporated in the diffuser or external thereof.

This type of diffuser can be used for perfuming the cockpit of a motor car: in this case the diffuser is arranged in a position in which it will be struck by a flow of air created by the car forced ventilation system. This flow causes a circulation of air in the cockpit thanks to which the vapours emitted through the permeable wall 2 will be drawn into the cockpit. In this case, too, by adjusting the regulating device the intensity of the emission of vapours from the volatile substance with be regulated.

In other embodiments, the regulating device, with which it is possible to vary the quantity of rising air flow striking the permeable wall 2, can comprise a wall, located in front of the permeable wall 2, and means for regulating the reciprocal distance between the walls; alternatively to the means for regulating a regulation valve can be fitted on the lower end of the two walls, to intercept the rising air flow to the inlet of the chamber defined between the walls. In a further embodiment the intensity of diffusion to the outside of the vapour is regulated by means of regulating an inflow of new air from the outside towards the inside of the diffuser. It is possible, for example, to open or close air vents on the frame of the diffuser. The above-described regulating devices represent some possible examples of means for regulating the air flow (activated for example by the flue effect) striking the permeable wall.

In the above-described embodiments, the container of the volatile substance can be provided with means for releasing the air, for example a release valve, to balance pressure within and without the container.

## Claims

1. A dispenser of airborne substances, comprising:
a closed body containing a volatile substance having at least one permeable wall (2);
a wall (10) facing the permeable wall (2) which delimits together with the permeable wall (2) a chamber (11) for a rising circulation of air, provided with a lower inlet (12) for air from a surrounding atmosphere and an upper outlet (13) for air mixed with vapours emitted through the permeable wall (2);
**characterised in that** it also comprises means (9) for
heating the chamber (11) in order that inside the chamber (11) a rising circulation of air is achieved by a flue effect; said means for heating being located inferiorly of the chamber (11).

2. The device of claim 1, comprising a regulating device (14) predisposed in front of the permeable wall (2) for regulating the circulation of air which strikes the permeable wall (2) through the chamber (11) going from the inlet (12) to the outlet (13).

3. The device of claim 2, **characterised in that** the regulating device (14) comprises at least one mobile switch (16) able selectively to assume at least one closed position, in which circulation of air to strike at least a part of the permeable wall (2) is prevented, and at least one open position in which air can strike the permeable wall (2).

4. The device of claim 3, **characterised in that** the switch (16) is rotatable on command about a hinge (15).

5. The device of claim **4**, **characterised in that** the switch (16) is brought into the at least one closed position by a cam (17), and returns elastically into the at least one open position.

6. The device of any one of claims from 2 to 5, **characterised in that** the regulating device (14) divides at least a part of the chamber (11) into two zones in which a first zone is facing and contiguous to the permeable wall (2), while a second zone is separated from the permeable wall (2), the first zone being provided with an inlet for air with an adjustable section of passage.

7. The device of any one of the preceding claims, **characterised in that** the permeable wall (2) is located in a vertical position, and the inlet (12) for external air is located at a lower height than the outlet of an air-vapour mixture.

## Patentansprüche

1. Spender von Luftschwebestoffen, enthaltend:
- einen geschlossenen Körper, der eine flüchtige Substanz enthält und wenigstens eine durchlässige Wand (2) hat;
- eine der durchlässigen Wand (2) zugewandte Wand (10), welche zusammen mit der durchlässigen Wand (2) eine Kammer (11) für einen ansteigenden Luftumlauf beschreibt, versehen mit einem unteren Einlass (12) für Luft aus einer umgebenden Atmosphäre und einem oberen Auslass (13) für mit Dämpfen vermischte Luft, die durch die durchlässige Wand (2) abgegeben werden;
**dadurch gekennzeichnet, dass** er ebenfalls Mittel (9) zum Erwärmen der Kammer (11) enthält, so dass im Inneren der Kammer (11) ein aufsteigender Luftumlauf durch eine Kaminwirkung erhalten wird, wobei die genannten Mittel zum Erwärmen unterhalb der Kammer (11) angeordnet sind.

2. Vorrichtung nach Patentanspruch 1, enthaltend eine Reguliervorrichtung (14), die vor der durchlässigen Wand (2) angeordnet ist, um den Luftumlauf zu regulieren, welcher die durchlässige Wand (2) streift, während er vom Einlass (12) zum Auslass (13) durch die Kammer (11) geht.

3. Vorrichtung nach Patentanspruch 2, **dadurch gekennzeichnet, dass** die Reguliervorrichtung (14) wenigstens einen beweglichen Umschalter (16) hat, in der Lage, wahlweise wenigstens eine geschlossene Position einzunehmen, in welcher das Streifen des Luftumlauf wenigstens an einem Teil der durchlässigen Wand (2) verhindert wird, und wenigstens eine offene Position, in welcher er die durchlässige Wand (2) streifen kann.

4. Vorrichtung nach Patentanspruch 3, **dadurch gekennzeichnet, dass** der Umschalter (16) auf eine Betätigung hin um ein Scharnier (15) gedreht werden kann.

5. Vorrichtung nach Patentanspruch 4, **dadurch gekennzeichnet, dass** der Umschalter (16) durch eine Nocke (17) in wenigstens eine geschlossene Position gebracht wird und elastisch in wenigstens eine offene Position zurück geht.

6. Vorrichtung nach einem jeden der Patentansprüche von 2 bis 5, **dadurch gekennzeichnet, dass** die Reguliervorrichtung (14) wenigstens einen Teil der Kammer (11) in zwei Bereiche teilt, von welchen ein erster Bereich der durchlässigen Wand (2) zugewandt ist und an diese angrenzt, während ein zweiter Bereich von der durchlässigen Wand (2) getrennt ist, wobei der erste Bereich mit einem Einlass für die Luft mit einer regulierbaren Durchlassweite versehen ist.

7. Vorrichtung nach einem beliebigen der vorstehenden Patentansprüche, **dadurch gekennzeichnet, dass** die durchlässige Wand (2) in einer vertikalen Position angeordnet ist, und dass der Einlass (12) für die Luft von aussen auf einer niedrigeren Höhe angeordnet ist als der Auslass für die Luft-Dampf Mischung.

## Revendications

1. Distributeur de substances en suspension dans l'air, comprenant:
un corps fermé contenant une substance volatile ayant au moins une paroi perméable (2);
une paroi (10) faisant face à la paroi perméable (2) et délimitant avec la paroi perméable (2) une chambre (11) pour la circulation vers le haut de l'air, pourvue d'une entrée inférieure (12) pour l'air provenant de l'atmosphère environnante et d'une sortie supérieure (13) pour l'air mélangé aux vapeurs émises au travers de la paroi perméable (2);
**caractérisé en ce qu'**il comprend également des moyens (9) de chauffage de la chambre (11) de manière à ce qu'à l'intérieur de la chambre (11) se produise une circulation vers le haut de l'air par effet de flux; lesdits moyens de chauffage étant disposés inférieurement à la chambre (11).

2. Distributeur selon la revendication 1, comprenant un dispositif de régulation (14) prédisposé en face de la paroi perméable (2) pour réguler la circulation de l'air qui frappe la paroi perméable (2) au travers de la chambre (11) en allant de l'entrée (12) à la sortie (13).

3. Distributeur selon la revendication 2, **caractérisé en ce que** le dispositif de régulation (14) comprend au moins un déflecteur mobile (16) pouvant sélectivement assumer au moins une position fermée, dans laquelle on empêche la circulation de l'air de frapper au moins une partie de la paroi perméable (2), et au moins une position ouverte dans laquelle l'air peut frapper la paroi perméable (2).

4. Distributeur selon la revendication 3, **caractérisé en ce que** le déflecteur (16) peut être pivoté sur demande autour d'un axe (15).

5. Distributeur selon la revendication 4, **caractérisé en ce que** le déflecteur (16) est amené dans ladite au moins une position fermée par une came (17), et retourne de manière élastique dans ladite au moins une position ouverte.

6. Distributeur selon n'importe laquelle des revendications 2 à 5, **caractérisé en ce que** le dispositif de régulation (14) divise au moins une partie de la chambre (11) en deux zones dans lesquelles une première zone fait face et est contiguë à la paroi perméable (2), alors qu'une seconde zone est séparée de la paroi perméable (2), la première zone étant pourvue d'une entrée pour l'air avec une section de passage réglable.

7. Distributeur selon n'importe laquelle des revendications précédentes, **caractérisé en ce que** la paroi perméable (2) est disposée dans une position verticale, et que l'entrée (12) pour l'air externe est disposée à une hauteur inférieure à celle de la sortie pour le mélange air-vapeur.
